# EUROPÄISCHE PATENTSCHRIFT

(11) **EP 3 870 123 B1**
(45) Veröffentlichungstag und Bekanntmachung des Hinweises auf die Patenterteilung: **25.06.2025**
(21) Anmeldenummer: 19779455.5
(22) Anmeldetag: 26.09.2019
(51) Int. Cl.: A61F 9/008, G02B 26/10

(54) **POSITIONIERUNGSEINRICHTUNG UND LICHTBEARBEITUNGSVORRICHTUNG MIT EINER SOLCHEN POSITIONIERUNGSEINRICHTUNG**
POSITIONING DEVICE AND LIGHT PROCESSOR HAVING SUCH A POSITIONING DEVICE
SYSTÈME DE POSITIONNEMENT ET DISPOSITIF DE TRAITEMENT À LA LUMIÈRE ÉQUIPÉ D'UN SYSTÈME DE POSITIONNEMENT DE CE TYPE

(30) Priorität: 23.10.2018 DE 102018218147
(43) Veröffentlichungstag der Anmeldung: 01.09.2021
(73) Patentinhaber: Physik Instrumente (PI) SE & Co. KG, 76228 Karlsruhe (DE)
(72) Erfinder: ROSENKRANZ, Mathias, 76593 Gernsbach (DE)
(74) Vertreter: Grünecker Patent- und Rechtsanwälte PartG mbB
(86) Internationale Anmeldenummer: PCT/EP2019/076046
(87) Internationale Veröffentlichungsnummer: WO 2020/083602

(56) Entgegenhaltungen:
- EP-A1- 2 363 742
- WO-A1-2012/035031
- CN-Y- 2 522 830
- US-A- 5 782 609
- US-A1- 2007 232 196

## Beschreibung

Die vorliegende Erfindung betrifft eine Positionierungseinrichtung zur Positionierung eines Objekts in einer Positionierungsebene und eine Lichtbearbeitungsvorrichtung mit einer solchen Positionierungseinrichtung. Eine solche Lichtbearbeitungsvorrichtung kann insbesondere zur Durchführung von augenlaserchirurgischen Behandlungen verwendet werden.

Der Begriff "Augenlasern" bezeichnet eine spezielle Augenoperation bzw. augenlaserchirurgische Behandlung, bei der durch Abtragen der Hornhaut eine brechungsbasierte Sehschwäche korrigiert werden kann. Bei derartigen Sehschwächen wird das Licht durch die Wölbung der Hornhaut und der Augenlinse abgelenkt und ins Augeninnere projiziert, wobei der Fokus nicht exakt auf der Fovea liegt, dem Ort des schärfsten Sehens auf der Netzhaut im Auge, sondern davor oder dahinter.

Beim "Augenlasern" wird zunächst wird eine hauchdünne Schicht der Hornhaut abgeschnitten und hochgeklappt, die dann dem anschließenden Schutz dient und die Heilung begünstigt. Anschließend trägt ein Laser die Oberfläche der Hornhaut ab, sodass sich die optischen Eigenschaften des Auges verändern und die Brechkraft der Hornhaut wieder optimal eingestellt wird.

Als Grauer Star (Katarakt) wird eine meist altersbedingte Eintrübung der natürlichen Linse des Auges bezeichnet, wobei die Lichtstrahlen im Augeninneren nicht mehr auf der Netzhaut fokussiert werden können, sodass die Licht- und Blendempfindlichkeit zunimmt, das Sehen verschwommen oder unscharf wird, teilweise werden Bilder doppelt gesehen oder Farben erscheinen grau. Bei der augenlaserchirurgischen Behandlung des Grauen Stars wird die getrübte natürliche Linse entfernt und durch eine klare Intraokularlinse ersetzt. Um an die getrübte Linse zu gelangen, wird mit einem Femtosekundenlaser ein kleiner Schnitt am Auge vorgenommen, wobei nur die vordere Linsenhülle kreisrund geöffnet und der in der sogenannten Hinterkammer des Auges angeordnete Linsenkern nach der Zerkleinerung abgesaugt wird. Die dann verbleibende Linsenhülle dient dann als "natürliche Halterung" für die neue Intraokularlinse.

Im Rahmen dieser augenlaserchirurgischen Behandlungen ist der Laserstrahl mit hoher Präzision auf einer vorgegebenen Spur bzw. Trajektorie über die Hornhaut des Patienten zu führen. Fehler bei der Positionierung und Einwirkdauer des Laserstrahls auf der Hornhaut des Patienten können nicht nur das Behandlungsergebnis nachteilig beeinflussen, sondern auch gravierende Schäden am Auge des Patienten hinterlassen.

Sollen beliebige Trajektorien bzw. Bewegungsbahnen in einer Ebene abgefahren werden, werden üblicherweise zwei Linearversteller (X und Y), welche rechtwinklig zueinander angeordnet sind und der Y-Versteller mit dem beweglichen Teil des X-Verstellers verbunden ist, eingesetzt. Durch ein sinusförmiges Geschwindigkeitsprofil der ersten Achse (X) und einem um 90° phasenverschobenen sinusförmigen Geschwindigkeitsprofil der zweiten Achse (Y), kann eine kreisförmige Bahnkurve realisiert werden. Bei einer kontinuierlichen Kreisbahnfahrt müssen beide Achsen (X und Y) jeweils zwei Mal die Richtung wechseln, d.h., der Vollkreis weist insgesamt vier Wendestellen auf. Aufgrund physikalischer Gegebenheiten (Trägheit, Umkehrspiel, endliche Sensorauflösung, endliche Reglergeschwindigkeit usw.) kommt es in den Wendepunkten zu einer gewissen Totzeit, d.h. die Richtungsänderung erfolgt nicht unendlich schnell, sondern es kommt zu einer Phase des Stillstands der jeweiligen Achse. Aufgrund dieser Totzeit ergibt sich ein dynamischer Positionsfehler (Schleppfehler), d.h. der Vollkreis weist insgesamt vier Fehlstellen auf.

Die Publikation FISHER Charles, [et al]: Cobra: A two-degree of freedom fiber optic positioning mechanism, IEEE Aerospace conference, IEEE, 2009, S. 1-11 offenbart eine weitere Positionierungsvorrichtung.

Das Dokument offenbart eine Schleifscheibe, umfassend: zwei Rotationsantriebe mit unterschiedlichen Durchmessern und eine Objektaufnahme zur Aufnahme des Objekts, wobei die Objektaufnahme an einen ersten der beiden Rotationsantriebe gekoppelt ist, der wiederum an den zweiten der beiden Rotationsantriebe gekoppelt ist, sodass die Objektaufnahme um die parallel und versetzt zueinander angeordneten Rotationsachsen der beiden Rotationsantriebe drehbar und somit in der Positionierungsebene verstellbar ist.

Der Erfindung liegt die Aufgabe zugrunde, eine Positionierungseinrichtung zur Positionierung eines Objekts in einer Positionierungsebene bereitzustellen, die den Positionsfehler (Schleppfehler) bei einer kontinuierlichen Kreisbahnfahrt im Gegensatz zu zwei rechtwinklig zueinander angeordneten Linearverstellern (X und Y) minimiert. Ferner liegt der Erfindung die Aufgabe zugrunde, eine Lichtbearbeitungsvorrichtung bereitzustellen, um beim Abfahren einer Kreisbahn mit dem Laserstrahl, insbesondere auf der Hornhaut eines Patienten, den von rechtwinklig angeordneten Linearverstellern (X und Y) bekannten dynamischen Positionsfehler (Schleppfehler) zu verringern. Zur Lösung dieser Aufgabe stellt die vorliegende Erfindung die Positionierungseinrichtung zur Positionierung eines Objekts in einer Positionierungsebene nach Anspruch 1 bereit, welche umfasst: zwei Rotationsantriebe mit unterschiedlichen Durchmessern und eine Objektaufnahme zur Aufnahme des Objekts, wobei die Objektaufnahme an einen ersten der beiden Rotationsantriebe gekoppelt ist, der wiederum an den zweiten der beiden Rotationsantriebe gekoppelt ist, sodass die Objektaufnahme um die parallel und versetzt zueinander angeordneten Rotationsachsen beider Rotationsantriebe drehbar und somit in der Positionierungsebene verstellbar ist. Durch die Erfindung wird das Abfahren einer beliebigen Trajektorie (Bewegungsbahn) im Allgemeinen, sowie das präzise Abfahren einer kreisförmigen Trajektorie im Speziellen ermöglicht. Durch die Erfindung sind keine fehlerbehafteten Wendestellen notwendig. Kreisförmige Trajektorien können kontinuierlich und präzise abgefahren werden. Durch das Fehlen von Wendepunkten und somit von Totzeiten kann der dynamische Positionsfehler bei Kreisbahnfahrten reduziert werden. Im Gegensatz zu einem einachsigen rotatorischen System können jedoch nicht nur Kreisbahnfahrten, sondern auch beliebige Trajektorien gefahren werden.

Der erste Rotationsantrieb und/oder der zweite Rotationsantrieb ist/sind ringförmig ausgebildet. Bei dieser Ausführung kann die Objektaufnahme innerhalb der Ringöffnung bzw. Apertur des ersten Rotationsantriebs angeordnet werden und die Posi- tionierungseinrichtung äußerst kompakt aufgebaut werden.

Vorteilhafte Weiterbildungen der Erfindung sind Gegenstände der Unteransprüche.

Es kann von Nutzen sein, wenn der erste Rotationsantrieb einen kleineren Durchmesser aufweist als der zweite Rotationsantrieb. Auf diese Weise ist die Position der Objektaufnahme besonders einfach zu steuern.

Es kann von Vorteil sein, wenn eine um die Rotationsachse des ersten Rotationsantriebs verlaufende Bewegungsbahn der Objektaufnahme die Rotationsachse des zweien Rotationsantriebs umschließt oder schneidet. Bei dieser Ausführungsform kann die Objektaufnahme durch entsprechende Betätigung der Rotationsantriebe zu jedem Punkt der Positionierungsebene innerhalb eines Kreises, der der Bewegungsbahn der Objektaufnahme um die Rotationsachse des zweiten Rotationsantriebs entspricht, verfahren werden.

Es kann nützlich sein, wenn der Durchmesser einer um die Rotationsachse des ersten Rotationsantriebs verlaufenden (ersten) Bewegungsbahn der Objektaufnahme wenigstens halb so groß ist wie der maximale Durchmesser einer um die Rotationsachse des zweiten Rotationsantriebs verlaufenden (zweiten) Bewegungsbahn der Objektaufnahme. Durch Verstellung des Abstands der Objektaufnahme von der Rotationsachse des zweiten Rotationsantriebs - mittels Betätigung des ersten Rotationsantriebs - kann der Durchmesser der zweiten Bewegungsbahn der Objektaufnahme - von null bis zum zweifachen Durchmesser der ersten Bewegungsbahn der Objektaufnahme - gezielt eingestellt werden.

Es kann sinnvoll sein, wenn die Drehrichtungen und/oder Drehgeschwindigkeiten der Rotationsantriebe gesondert voneinander steuerbar sind. Dadurch kann die Objektaufnahme im Prinzip jede Trajektorie innerhalb der Positionierungsebene und innerhalb der zweiten Bewegungsbahn der Objektaufnahme um die Rotationsachse des zweiten Rotationsantriebs abfahren. Vorzugsweise ist/sind der erste Rotationsantrieb und/oder der zweite Rotationsantrieb als piezoelektrischer Rotationsantrieb ausgebildet.

Es kann aber auch hilfreich sein, wenn jeder ringförmige Rotationsantrieb zwei relativ zueinander drehbare Ringe aufweist, von denen einer als Statorring und der andere als Rotorring ausgebildet ist. Diese Ausführungsform erweist sich als besonders kompakt und ist kostengünstig herstellbar.

Es kann sich auch als nützlich erweisen, wenn der Statorring des ersten Rotationsantriebs drehfest an den Rotorring des zweiten Rotationsantriebs gekoppelt ist, vorzugsweise über eine lösbare Kupplung.

Es kann sinnvoll sein, wenn die Objektaufnahme an einem Innenumfang des (Rotorrings des) ersten Rotationsantriebs angeordnet ist. Auch diese Ausführungsform erweist sich als besonders kompakt.

Ein weiterer Aspekt der vorliegenden Erfindung betrifft eine Lichtbearbeitungsvorrichtung nach Anspruch 10, umfassend eine Positionierungseinrichtung nach einem der vorangehenden Ansprüche sowie ein Lichtlenkungselement, das an oder in der Objektaufnahme der Positionierungseinrichtung angeordnet ist und mittels der Positionierungseinrichtung in der Positionierungsebene verstellbar ist, um einen Lichtstrahl, insbesondere einen Laserstrahl, auf ein durch den Lichtstrahl zu bearbeitendes Objekt zu lenken. Mit der Lichtbearbeitungsvorrichtung kann ein Laserstrahl besonders präzise entlang von Kreisbahnen auf ein zu bearbeitendes Objekt bzw. über dessen Oberfläche gelenkt werden. Beispielsweise sind für augenlaserchirurgische Behandlungen sehr leistungsstarke Laser erforderlich, die ein hohes Gewicht aufweisen und ein großes Volumen einnehmen. Unabhängig von derartigen Einschränkungen ermöglicht die erfindungsgemäße Lichtbearbeitungsvorrichtung eine besonders schonende und sichere augenlaserchirurgische Behandlung des Grauen Stars oder von brechungsbasierten Sehschwächen durch Veränderung der Hornhautkrümmung eines Patienten, weil lediglich das Lichtlenkungselement bewegt werden muss, um den Laserstrahl abzulenken. Das Lichtlenkungselement kann beispielsweise als Spiegel, Prisma, Lichtleiter, Linse, Objektiv oder dergleichen ausgebildet sein.

Es kann ebenfalls von Vorteil sein, wenn das Lichtlenkungselement an oder in der Objektaufnahme der Positionierungseinrichtung derart angeordnet und/oder ausrichtbar ist, dass der Lichtstrahl parallel zu den Rotationsachsen beider Rotationsantriebe verläuft. Bei dieser Ausführungsform ist eine besonders präzise Ausrichtung des Lichtstrahls möglich, was das Ergebnis der Bearbeitung begünstigt.

Es kann sich als nützlich erweisen, wenn der Lichtstrahl durch die Ringöffnung des ersten Rotationsantriebs und/oder durch die Ringöffnung des zweiten Rotationsantriebs verläuft. Diese Ausführungsform erlaubt es beispielsweise, Strahlengänge durch das System hindurch zu leiten.

Es kann auch sinnvoll sein, wenn das Lichtlenkungselement der laserlichtemittierende Teil eines Lasers, vorzugsweise eines Femtosekundenlasers ist. Ein derartiger Laser ist besonders zur Behandlung des Grauen Stars oder von brechungsbasierten Sehschwächen geeignet.

Ein weiterer Aspekt betrifft die Verwendung der Lichtbearbeitungsvorrichtung nach einem der Ansprüche 10 bis 13 zur Behandlung des Grauen Stars oder einer brechungsbasierten Sehschwäche durch Einwirkung auf die Hornhaut eines Patienten mittels des Lichtstrahls bzw. Laserstrahls.

Noch ein weiterer Aspekt betrifft ein Verfahren zur augenlaserchirurgischen Behandlung unter Verwendung der Lichtbearbeitungsvorrichtung nach einem der Ansprüche 10 bis 13, umfassend die Schritte:
- Anordnen der Lichtbearbeitungsvorrichtung in einem Abstand von der Hornhaut eines Patienten, vorzugsweise derart, dass die Positionierungsebene der Positionierungseinrichtung exakt oder im Wesentlichen senkrecht zu einer Normalen auf die Hornhaut des Patienten ausgerichtet ist;
- Verstellen des in der Objektaufnahme der Positionierungseinrichtung angeordneten Lichtlenkungselements in der Positionierungsebene mittels des ersten Rotationsantriebs und/oder des zweiten Rotationsantriebs, sodass der vom Lichtlenkungselement gelenkte Lichtstrahl eine zumindest abschnittsweise kreisförmige und/oder eine zumindest abschnittsweise bogenförmige Spur und/oder eine zumindest abschnittsweise gerade Spur auf der Hornhaut des Patienten abfährt, zur Veränderung der Hornhautkrümmung des Patienten.

Es kann sich als nützlich erweisen, die Positionierungseinrichtung mit einem oder mehreren Linearantrieben bzw. Linearverstellern zu kombinieren, um das Abfahren linearer Trajektorien zu erleichtern.

Weitere bevorzugte Ausführungsformen ergeben sich durch Kombinationen der in den Ansprüchen, den Zeichnungen und in der Beschreibung offenbarten Merkmale.

### Begriffe und Definitionen

### Bewegungsbahn

Die Bewegungsbahn der Objektaufnahme um die Rotationsachse des Rotationsantriebs entspricht dem imaginären Kreis, den die Objektaufnahme bei einer vollständigen Umdrehung um die Rotationsachse des Rotationsantriebs beschreibt.

### Objektaufnahme

Der Begriff Objektaufnahme bezeichnet eine Aufnahme oder Halterung für ein von der Positionierungseinrichtung zu bewegendes Objekt.

### Positionierungsebene

Als Positionierungsebene wird die Ebene bezeichnet, in der die Objektaufnahme durch Betätigung der Rotationsantriebe bewegbar ist. Die Positionierungsebene erstreckt sich senkrecht zu den Rotationsachsen beider Rotationsantriebe.

Lichtlenkungselement Als Lichtlenkungselement wird ein Element bezeichnet, das in der Lage ist, einen Lichtstrahl zu lenken. Vorzugsweise ist das Lichtlenkungselement als Spiegel, Prisma, Linse, Lichtleiter, Objektiv oder dergleichen ausgebildet.

### Kurze Beschreibung der Figuren

Es zeigen:
Fig. 1 eine schematische und perspektivische Ansicht einer erfindungsgemäßen Lichtbearbeitungsvorrichtung mit einer erfindungsgemäßen Positionierungseinrichtung, die zwei ringförmige Rotationsantriebe mit unterschiedlichen Durchmessern aufweist.
Figur 2 eine schematische und perspektivische Ansicht der Lichtbearbeitungsvorrichtung gemäß Figur 1 bei einer augenlaserchirurgischen Behandlung, wobei ein Lichtlenkungselement durch die Positionierungseinrichtung mittels Betätigung der beiden Rotationsantriebe in der Positionierungsebene verstellt wird, um den vom Lichtlenkungselement gelenkten Lichtstrahl entlang einer zumindest abschnittsweise kreisförmigen Trajektorie über die Hornhaut eines Patienten zu führen.
Fig. 3 eine schematische und perspektivische Ansicht ähnlich der Figur 1.
Fig. 4 eine schematische und perspektivische Ansicht ähnlich der Figur 2 aus anderem Blickwinkel.
Fig. 5 in den Ansichten (a) bis (d) verschiedene Stadien einer augenlaserchirurgischen Behandlung unter Verwendung der Lichtbearbeitungsvorrichtung gemäß einer der Figuren 1 bis 4.
Figur 6 eine schematische Draufsicht entlang der Rotationsachsen beider Rotationsantriebe senkrecht auf die Bewegungsbahnen der Objektaufnahme um jede der beiden Rotationsachsen gemäß einer bevorzugten Ausführungsform der erfindungsgemäßen Positionierungseinrichtung.

### Detaillierte Beschreibung des bevorzugten Ausführungsbeispiels

Das bevorzugte Ausführungsbeispiel der Erfindung, das nachstehend mit Bezug auf die beiliegenden Figuren im Detail beschrieben wird, betrifft eine Lichtbearbeitungsvorrichtung in Gestalt eines augenlaserchirurgischen Instruments, umfassend ein Lichtlenkungselement in Form eines Spiegels oder Lichtleiters und eine erfindungsgemäße Positionierungseinrichtung 1 mit zwei Rotationsantrieben 2, 3 unterschiedlichen Durchmessers und einer Objektaufnahme 4, in welcher das Lichtlenkungselement angeordnet ist. Dieses Lichtlenkungselement ist ausgebildet, um einen Laserstrahl 5 zur augenlaserchirurgischen Behandlung auf die Hornhaut 6 eines Patienten zu lenken, wobei das Lichtlenkungselement mittels der Positionierungseinrichtung 1 in der Positionierungsebene verstellbar ist. Das Lichtlenkungselement ist im vorliegenden Fall der lichtemittierende Teil eines Femtosekundenlasers zur Durchführung einer lasergestützten Augenoperation.

Die erfindungsgemäße Positionierungseinrichtung 1 dient zur Positionierung des Lichtlenkungselements in einer Positionierungsebene. Die das Lichtlenkungselement aufnehmende Objektaufnahme 4 ist an den ersten Rotationsantrieb 3 mit kleinerem Durchmesser gekoppelt, der wiederum vorzugsweise über eine Kupplung 23 lösbar an den zweiten Rotationsantrieb 2 mit größerem Durchmesser gekoppelt ist, sodass die Objektaufnahme 4 um die parallel und versetzt zueinander angeordneten Rotationsachsen A2, A3 beider Rotationsantriebe 2, 3 drehbar und somit in der Positionierungsebene verstellbar ist.

Wie in Figur 6 dargestellt ist, schneidet eine um die Rotationsachse A3 des ersten Rotationsantriebs 3 verlaufende Bewegungsbahn B3 der Objektaufnahme 4 die Rotationsachse A2 des zweiten Rotationsantriebs 2. Demnach ist der Durchmesser der um die Rotationsachse A3 des ersten Rotationsantriebs 3 verlaufenden Bewegungsbahn B3 der Objektaufnahme 4 exakt halb so groß wie der maximale Durchmesser einer um die Rotationsachse A2 des zweiten Rotationsantriebs 2 verlaufenden Bewegungsbahn B2 der Objektaufnahme 4. D. h., dass die Objektaufnahme 4 bzw. das daran angeordnete Lichtlenkungselement in der Positionierungsebene jede Position erreichen kann, die sich innerhalb der um die Rotationsachse A2 des Rotationsantriebs 2 mit größerem Durchmesser verlaufenden Bewegungsbahn B2 der Objektaufnahme 4 befindet.

Weil die Drehrichtungen und Drehgeschwindigkeiten der Rotationsantriebe 2, 3 gesondert voneinander steuerbar sind, kann die Objektaufnahme 4 beliebige Trajektorien innerhalb der Positionierungsebene abfahren, insbesondere auch kreisförmige, bogenförmige oder gerade Abschnitte davon.

In der vorliegenden Ausführungsform ist jeder der beiden Rotationsantriebe 2, 3 ringförmig ausgebildet und weist zwei relativ zueinander drehbare Ringe 21, 31; 22, 32 auf, von denen einer als Statorring 21, 31 und der andere als Rotorring 22, 32 ausgebildet ist. Der Statorring 31 des ersten Rotationsantriebs 3 ist vorzugsweise über die lösbare Kupplung 23 drehfest an den Rotorring 22 des zweiten Rotationsantriebs 2 gekoppelt und die Objektaufnahme 4 ist an einem Innenumfang des Rotorrings 32 des ersten Rotationsantriebs 3 angeordnet.

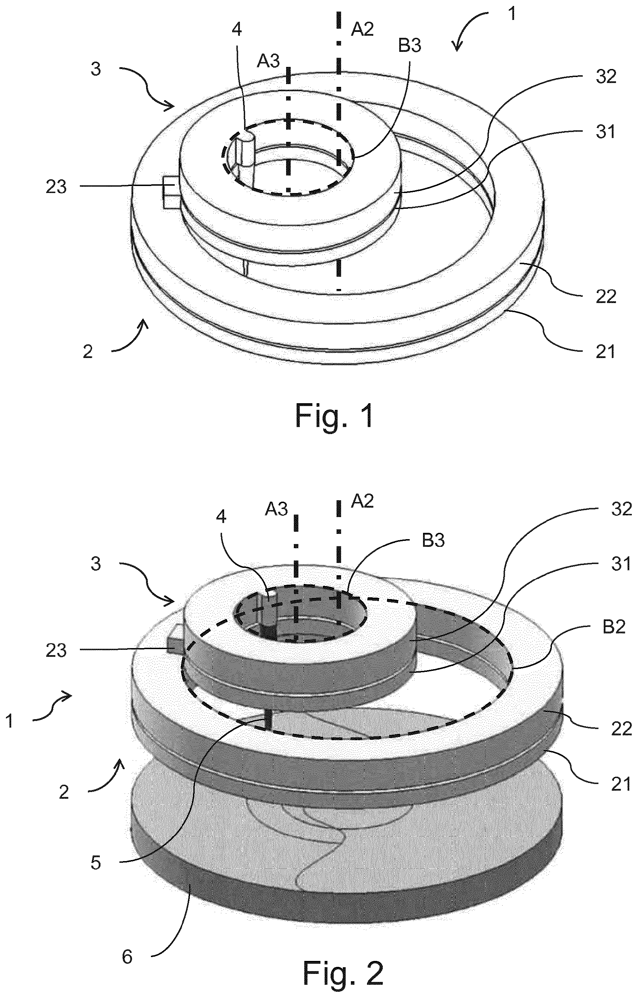

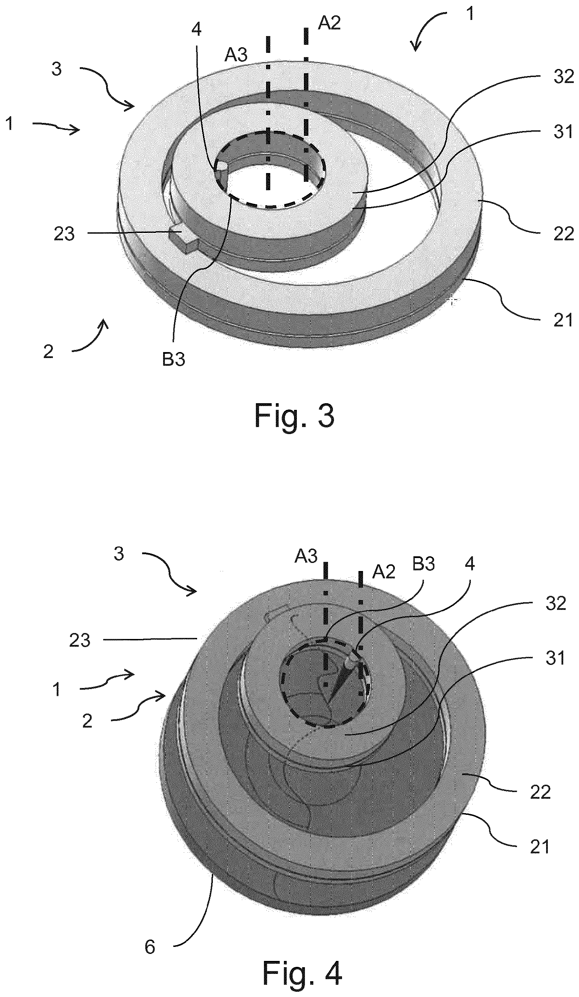

Das Lichtlenkungselement ist in der Objektaufnahme 4 der Positionierungseinrichtung 1 derart angeordnet, dass der Laserstrahl 5 parallel zu den Rotationsachsen A2, A3 der beider Rotationsantriebe 2, 3 verläuft. Dabei wird der Laserstrahl 5 durch die Ringöffnung beider Rotationsantriebe geführt, um im Betriebszustand auf einem darunter angeordneten Zielobjekt 6 aufzutreffen (vgl. Fig. 2, 4, 5).

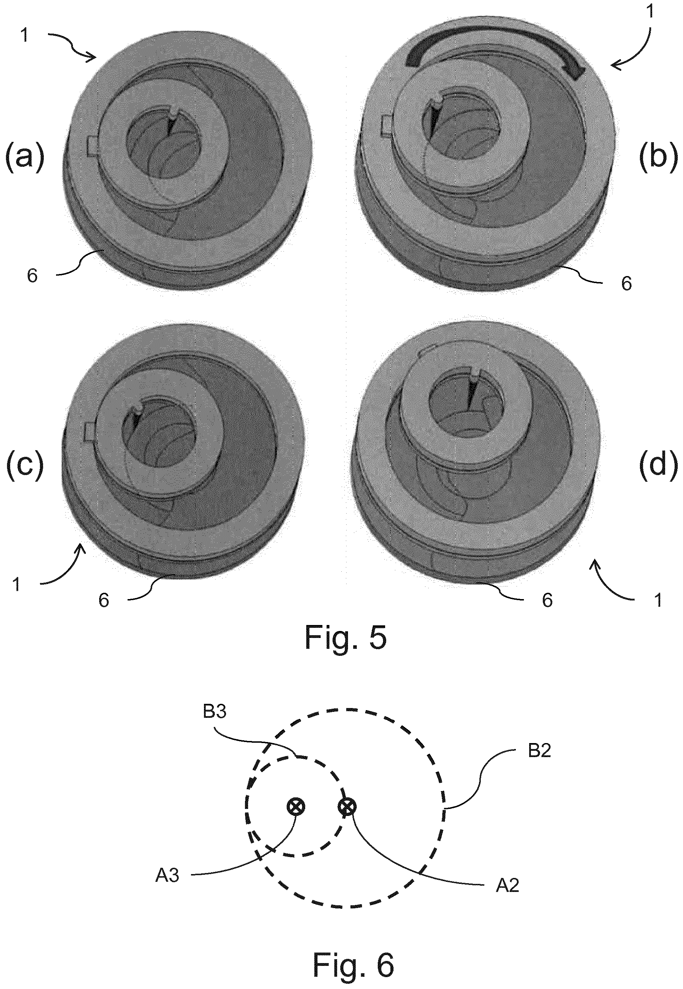

Gemäß einem Verfahren zur augenlaserchirurgischen Behandlung wird die erfindungsgemäße Lichtbearbeitungsvorrichtung in einem Abstand von der Hornhaut 6 eines Patienten angeordnet, sodass die Positionierungsebene der Positionierungseinrichtung 1 idealerweise exakt oder im Wesentlichen senkrecht zu einer Normalen auf die Hornhaut 6 des Patienten ausgerichtet ist (vgl. Fig. 2, 4, 5). Anschließend wird das in der Objektaufnahme 4 der Positionierungseinrichtung 1 angeordnete Lichtlenkungselement durch Betätigung beider Rotationsantriebe in der Positionierungsebene verstellt, sodass der von dem Lichtlenkungselement gelenkte Laserstrahl 5 auf der Hornhaut 6 des Patienten eine Spur abfährt, die kreisförmige, bogenförmige oder gerade Streckenabschnitte enthalten kann, um die Hornhautkrümmung des Patienten zu verändern oder Schnitte in die Hornhaut des Patienten einzubringen. Einzelne Stadien dieses Prozesses sind in den Ansichten (a) bis (d) der Fig. 5 dargestellt.

Durch Rotation bzw. Verstellung des ersten Rotationsantriebs 3 können verschiedene Kreisdurchmesser gezielt eingestellt werden. Durch Rotation bzw. Verstellung des zweiten Rotationsantriebs 2 können Kreisbahnen mit dem zuvor eingestellten Kreisdurchmesser kontinuierlich abgefahren werden. Durch die kombinierte Rotation von erstem und zweitem Rotationsantrieb 2, 3 kann jede beliebige Trajektorie abgefahren werden.

### Bezugszeichenliste

- 1: Positionierungseinrichtung
- 2: Zweiter Rotationsantrieb (großer Durchmesser)
- 3: Erster Rotationsantrieb (kleiner Durchmesser)
- 4: Objektaufnahme
- 5: Strahlengang bzw. Lichtstrahl bzw. Laserstrahl
- 6: Zielobjekt bzw. Hornhaut
- 21: Statorring des zweiten Rotationsantriebs (großer Durchmesser)
- 22: Rotorring des zweiten Rotationsantriebs (großer Durchmesser)
- 23: Kupplungsabschnitt
- 31: Statorring des ersten Rotationsantriebs (kleiner Durchmesser)
- 32: Rotorring des ersten Rotationsantriebs (kleiner Durchmesser)
- A2: Rotationsachse des zweiten Rotationsantriebs (großer Durchmesser)
- A3: Rotationsachse des ersten Rotationsantriebs (kleiner Durchmesser)
- B2: Bewegungsbahn der Objektaufnahme um die Rotationsachse A2
- B3: Bewegungsbahn der Objektaufnahme um die Rotationsachse A3

## Patentansprüche

1. Positionierungseinrichtung (1) zur Positionierung eines Objekts in einer Positionierungsebene, umfassend: zwei Rotationsantriebe (2, 3) mit unterschiedlichen Durchmessern und eine Objektaufnahme (4) zur Aufnahme des Objekts, wobei die Objektaufnahme (4) an einen ersten der beiden Rotationsantriebe (3) gekoppelt ist, der wiederum an den zweiten der beiden Rotationsantriebe (2) gekoppelt ist, sodass die Objektaufnahme (4) um die parallel und versetzt zueinander angeordneten Rotationsachsen (A2, A3) der beiden Rotationsantriebe (2, 3) drehbar und somit in der Positionierungsebene verstellbar ist, **dadurch gekennzeichnet, dass** der erste Rotationsantrieb (3) und/oder der zweite Rotationsantrieb (2) ringförmig ausgebildet ist/sind.

2. Positionierungseinrichtung (1) nach Anspruch 1, **dadurch gekennzeichnet, dass** der erste Rotationsantrieb (3) einen kleineren Durchmesser aufweist als der zweite Rotationsantrieb (2).

3. Positionierungseinrichtung (1) nach einem der vorangehenden Ansprüche, **dadurch gekennzeichnet, dass** eine um die Rotationsachse (A3) des ersten Rotationsantriebs (3) verlaufende Bewegungsbahn (B3) der Objektaufnahme (4) die Rotationsachse (A2) des zweiten Rotationsantriebs (2) umschließt oder schneidet.

4. Positionierungseinrichtung (1) nach einem der vorangehenden Ansprüche, **dadurch gekennzeichnet, dass** der Durchmesser einer um die Rotationsachse (A3) des ersten Rotationsantriebs (3) verlaufenden Bewegungsbahn (B3) der Objektaufnahme (4) wenigstens halb so groß ist wie der maximale Durchmesser einer um die Rotationsachse (A2) des zweiten Rotationsantriebs (2) verlaufenden Bewegungsbahn (B2) der Objektaufnahme (4).

5. Positionierungseinrichtung (1) nach einem der vorangehenden Ansprüche, **dadurch gekennzeichnet, dass** die Drehrichtungen und/oder Drehgeschwindigkeiten der Rotationsantriebe (2, 3) gesondert voneinander steuerbar sind.

6. Positionierungseinrichtung (1) nach einem der vorangehenden Ansprüche, **dadurch gekennzeichnet, dass** jeder ringförmige Rotationsantrieb (2, 3) zwei relativ zueinander drehbare Ringe aufweist (21, 31; 22, 32), von denen einer als Statorring (21, 31) und der andere als Rotorring (22, 32) ausgebildet ist.

7. Positionierungseinrichtung (1) nach einem der vorangehenden Ansprüche, **dadurch gekennzeichnet, dass** der Statorring (31) des ersten Rotationsantriebs (3) drehfest an den Rotorring (22) des zweiten Rotationsantriebs (2) gekoppelt ist, vorzugsweise über eine lösbare Kupplung (23).

8. Positionierungseinrichtung (1) nach einem der vorangehenden Ansprüche, **dadurch gekennzeichnet, dass** die Objektaufnahme (4) an einem Innenumfang des ersten Rotationsantriebs (3) angeordnet ist.

9. Lichtbearbeitungsvorrichtung, umfassend eine Positionierungseinrichtung (1) nach einem der vorangehenden Ansprüche sowie ein Lichtlenkungselement, das an der Objektaufnahme (4) der Positionierungseinrichtung (1) angeordnet ist und mittels der Positionierungseinrichtung (1) in der Positionierungsebene verstellbar ist, um einen Lichtstrahl (5) auf ein durch den Lichtstrahl (5) zu bearbeitendes Objekt zu lenken.

10. Lichtbearbeitungsvorrichtung nach Anspruch 9, **dadurch gekennzeichnet, dass** das Lichtlenkungselement an der Objektaufnahme (4) der Positionierungseinrichtung (1) derart angeordnet und/oder ausrichtbar ist, dass der Lichtstrahl (5) parallel zu den Rotationsachsen (A2, A3) beider Rotationsantriebe (2, 3) verläuft.

11. Lichtbearbeitungsvorrichtung nach einem der Ansprüche 9 oder 10 in Kombination mit einem der der Ansprüche 6 bis 9, **dadurch gekennzeichnet, dass** der Lichtstrahl (5) durch die Ringöffnung des ersten Rotationsantriebs (3) und/oder durch die Ringöffnung des zweiten Rotationsantriebs (2) geführt wird.

12. Lichtbearbeitungsvorrichtung nach einem der Ansprüche 9 bis 11, **dadurch gekennzeichnet, dass** das Lichtlenkungselement der laserlichtemittierende Teil eines Lasers, vorzugsweise eines Femtosekundenlasers ist.

## Claims

1. Positioning device (1) for positioning an object in a positioning plane, comprising: two rotation drives (2, 3) having different diameters and an object receiver (4) for receiving said object, where said object receiver (4) is coupled to a first of said two rotation drives (3), which in turn is coupled to the second of said two rotation drives (2), so that said object receiver (4) can be rotated about the axes of rotation (A2, A3) of both rotation drives (2, 3), that are arranged parallel and offset from one another, and is thereby adjustable in said positioning plane, **characterized in that** said first rotation drive (3) and/or said second rotation drive (2) is/are ring-shaped.

2. Positioning device (1) according to claim 1, **characterized in that** said first rotation drive (3) has a smaller diameter than said second rotation drive (2).

3. Positioning device (1) according to one of the preceding claims, **characterized in that** a path of motion (B3) of said object receiver (4) extending around said axis of rotation (A3) of said first rotation drive (3) encloses or intersects said axis of rotation (A2) of said second rotation drive (2).

4. Positioning device (1) according to one of the preceding claims, **characterized in that** the diameter of a path of motion (B3) of said object receiver (4) extending around said axis of rotation (A3) of said first rotation drive (3) is at least half as large as the maximum diameter of a path of motion (B2) of said object receiver (4) extending around said axis of rotation (A2) of said second rotation drive (2).

5. Positioning device (1) according to one of the preceding claims, **characterized in that** the directions of rotation and/or speeds of rotation of said rotation drives (2, 3) can be controlled separately from one another.

6. Positioning device (1) according to one of the preceding claims, **characterized in that** each ring-shaped rotation drive (2, 3) comprises two rings (21, 31; 22, 32) rotatable relative to one another, one of which is formed as a stator ring (21, 31) and the other as a rotor ring (22, 32).

7. Positioning device (1) according to one of the preceding claims, **characterized in that** said stator ring (31) of said first rotation drive (3) is coupled in a rotationally fixed manner to said rotor ring (22) of said second rotation drive (2), preferably by way of a releasable coupling (23).

8. Positioning device (1) according to one of the preceding claims, **characterized in that** said object receiver (4) is arranged on an inner circumference of said first rotation drive (3).

9. Light processor comprising a positioning device (1) according to one of the preceding claims as well as a light-directing element which is arranged on said object receiver (4) of said positioning device (1) and can be adjusted in said positioning plane by way of said positioning device (1) in order to direct a light beam (5) onto an object to be processed by said light beam (5).

10. Light processor according to claim 9, **characterized in that** said light-directing element is arranged and/or alignable on said object receiver (4) of said positioning device (1) such that said light beam (5) extends parallel to said axes of rotation (A2, A3) of both rotation drives (2, 3).

11. Light processor according to one of the claims 9 or 10 in combination with one of the claims 6 to 9, **characterized in that** said light beam (5) is guided through the ring opening of said first rotation drive (3) and/or through the ring opening of said second rotation drive (2).

12. Light processor according to one of the claims 9 to 11, **characterized in that** said light-directing element is the laser light-emitting part of a laser, preferably a femtosecond laser.

## Revendications

1. Dispositif de positionnement (1) permettant de positionner un objet dans un plan de positionnement, comprenant : deux entraînements rotatifs (2, 3) de différents diamètres et un logement d'objet (4) permettant d'accueillir l'objet, dans lequel le logement d'objet (4) est couplé à un premier des deux entraînements rotatifs (3), qui est à son tour couplé au deuxième des deux entraînements rotatifs (2), de sorte que le logement d'objet (4) peut tourner autour des axes de rotation (A2, A3) des deux entraînements rotatifs (2, 3) agencés de manière parallèle et décalée l'un par rapport à l'autre et peut ainsi être ajusté dans le plan de positionnement, **caractérisé en ce que** le premier entraînement rotatif (3) et/ou le deuxième entraînement rotatif (2) est/sont de forme annulaire.

2. Dispositif de positionnement (1) selon la revendication 1, **caractérisé en ce que** le premier entraînement rotatif (3) présente un diamètre plus petit que celui du deuxième entraînement rotatif (2).

3. Dispositif de positionnement (1) selon l'une quelconque des revendications précédentes, **caractérisé en ce qu'une** trajectoire de déplacement (B3) du logement d'objet (4) s'étendant autour de l'axe de rotation (A3) du premier entraînement rotatif (3) entoure ou coupe l'axe de rotation (A2) du deuxième entraînement rotatif (2).

4. Dispositif de positionnement (1) selon l'une quelconque des revendications précédentes, **caractérisé en ce que** le diamètre d'une trajectoire de déplacement (B3), s'étendant autour de l'axe de rotation (A3) du premier entraînement rotatif (3), du logement d'objet (4) représente au moins la moitié du diamètre maximal d'une trajectoire de déplacement (B2), s'étendant autour de l'axe de rotation (A2) du deuxième entraînement rotatif (2), du logement d'objet (4).

5. Dispositif de positionnement (1) selon l'une quelconque des revendications précédentes, **caractérisé en ce que** les sens de rotation et/ou les vitesses de rotation des entraînements rotatifs (2, 3) peuvent être commandés séparément les un(e)s des autres.

6. Dispositif de positionnement (1) selon l'une quelconque des revendications précédentes, **caractérisé en ce que** chaque entraînement rotatif (2, 3) annulaire présente deux bagues (21, 31 ; 22, 32) rotatives l'une par rapport à l'autre, dont l'une est réalisée sous la forme d'une bague de stator (21, 31) et l'autre sous la forme d'une bague de rotor (22, 32).

7. Dispositif de positionnement (1) selon l'une quelconque des revendications précédentes, **caractérisé en ce que** la bague de stator (31) du premier entraînement rotatif (3) est couplée de manière solidaire en rotation à la bague de rotor (22) du deuxième entraînement rotatif (2), de manière préférée par l'intermédiaire d'un couplage amovible (23).

8. Dispositif de positionnement (1) selon l'une quelconque des revendications précédentes, **caractérisé en ce que** le logement d'objet (4) est agencé au niveau d'une périphérie interne du premier entraînement rotatif (3).

9. Dispositif de traitement de lumière, comprenant un dispositif de positionnement (1) selon l'une quelconque des revendications précédentes, ainsi qu'un élément de direction de lumière qui est agencé au niveau du logement d'objet (4) du dispositif de positionnement (1) et qui peut être ajusté dans le plan de positionnement au moyen du dispositif de positionnement (1) afin de diriger un faisceau lumineux (5) sur un objet à traiter grâce au faisceau lumineux (5).

10. Dispositif de traitement de lumière selon la revendication 9, **caractérisé en ce que** l'élément de direction de lumière est agencé et/ou peut être orienté sur le logement d'objet (4) du dispositif de positionnement (1) de telle manière que le faisceau lumineux (5) s'étend parallèlement aux axes de rotation (A2, A3) des deux entraînements rotatifs (2, 3).

11. Dispositif de traitement de lumière selon l'une quelconque des revendications 9 ou 10, en combinaison avec l'une quelconque des revendications 6 à 9, **caractérisé en ce que** le faisceau lumineux (5) est guidé à travers l'orifice annulaire du premier entraînement rotatif (3) et/ou à travers l'orifice annulaire du second entraînement rotatif (2).

12. Dispositif de traitement de lumière selon l'une quelconque des revendications 9 à 11, **caractérisé en ce que** l'élément de direction de lumière est la partie émettrice de lumière laser d'un laser, de manière préférée d'un laser femtoseconde.
